# FASCICULE DE BREVET EUROPEEN

(11) **EP 2 755 706 B1**
(45) Date de publication et mention de la délivrance du brevet: **16.11.2016**
(21) Numéro de dépôt: 12756489.6
(22) Date de dépôt: 11.09.2012
(51) Int. Cl.: A61M 15/00, A61M 16/00, A61M 16/14

(54) **CHAMBRE D'INHALATION DESTINÉE À ÊTRE INTÉGRÉE SUR UN CIRCUIT D'UN DISPOSITIF DE RESPIRATION EN VENTILATION MECANIQUE**
INHALATIONSKAMMER ZUM EINBAU IN DEN KREISLAUF EINER RESPIRATORISCHEN VORRICHTUNG MIT MECHANISCHER BEATMUNG
INHALATION CHAMBER TO BE BUILT INTO A CIRCUIT OF A MECHANICAL-VENTILATION RESPIRATORY DEVICE

(30) Priorité: 15.09.2011 FR 1158227
(43) Date de publication de la demande: 23.07.2014
(73) Titulaire: Protecsom, 50700 Valognes (FR); Université François-Rabelais de Tours, 37020 Tours Cedex 1 (FR)
(72) Inventeur: VECELLIO-NONE, Laurent, F-37170 Chambray Les Tours (FR)
(74) Mandataire: Le Guen-Maillet
(86) Numéro de dépôt international: PCT/EP2012/067715
(87) Numéro de publication internationale: WO 2013/037759

(56) Documents cités:
- WO-A1-2005/102427
- WO-A1-2010/100557
- US-A1- 2007 283 954
- US-A1- 2008 264 412
- US-B1- 6 550 476
- US-B1- 6 962 152

## Description

La présente invention se situe dans le domaine des dispositifs de ventilation mécanique, notamment des dispositifs permettant l'introduction de médicaments dans le flux d'air généré par le dispositif de ventilation à destination des voies respiratoires d'un patient par l'intermédiaire d'un aérosol-doseur et/ou d'un nébuliseur. Plus précisément, l'invention concerne une chambre d'inhalation destinée à être intégrée à un circuit d'un dispositif de respiration en ventilation mécanique, ainsi qu'un tel dispositif.

En ventilation mécanique, la délivrance de médicaments par voie aérosol est une voie d'administration de plus en plus utilisée, en particulier chez les patients souffrants de Broncho Pneumopathie Chronique Obstructive (BPCO), d'asthme ou d'insuffisance respiratoire chronique obstructive. Par exemple, les broncho-dilatateurs sont des médicaments prescrits en aérosol au cours d'une ventilation mécanique. D'autres classes médicamenteuses sont aussi administrées sous forme d'aérosol. A ce titre, les antibiotiques à fortes doses représentent une nouvelle voie de recherche dans le traitement des infections pulmonaires graves. La ventilation mécanique crée une situation particulière dans la genèse et le transport de l'aérosol pour atteindre le parenchyme pulmonaire.

Dans un aérosol-doseur ou pMDI (acronyme anglais signifiant pressurized Metered Dose Inhaler) le médicament se trouve en suspension dans un liquide et peut être mis sous pression à l'aide d'un gaz propulseur. Les aérosols doseurs représentent le système de génération d'aérosol probablement le plus adapté pour administrer certains médicaments tels que les bêtas 2 mimétiques ou les anti-cholinergiques en ventilation mécanique dans le traitement des syndromes obstructifs. Un inconvénient lié à l'utilisation des aérosols-doseurs est la synchronisation nécessaire de l'administration de l'aérosol avec l'inspiration de l'utilisateur. En ventilation mécanique, la synchronisation de l'administration doit se faire également avec la première partie de la phase inspiratoire du respirateur. L'utilisation d'une chambre d'inhalation permet d'améliorer l'efficacité de l'aérosol-doseur.

Les chambres d'inhalation fonctionnent comme des volumes de freinage où les particules éjectées de l'aérosol doseur peuvent être ralenties par les forces de frottement. Avec une chambre d'inhalation, le dépôt pulmonaire est quatre à six fois supérieur à celui obtenu sans chambre d'inhalation. La position de l'aérosol doseur et de sa chambre permet un dépôt pulmonaire optimal quand il est placé à 15 cm de la sonde d'intubation.

L'intérêt des nébuliseurs repose sur la possibilité d'administrer toute sorte de médicaments liquides, c'est-à-dire non seulement des broncho-dilatateurs mais également d'autres médicaments non disponibles sous la forme d'aérosol doseur, tels que des antibiotiques. Parmi les différents types de nébuliseurs actuellement commercialisés, le nébuliseur à tamis vibrant est reconnu comme le plus performant. Il s'agit d'une nouvelle génération de nébuliseurs conçus pour la ventilation mécanique. Ce nébuliseur permet de nébuliser la totalité du volume introduit dans le réservoir sans augmentation importante de la température de la solution. Son ergonomie et son faible encombrement en font actuellement un système de prédilection pour la nébulisation en ventilation mécanique.

Généralement, lorsque l'utilisation d'un nébuliseur et d'un aérosol-doseur est préconisée, l'aérosol-doseur est connecté à une chambre d'inhalation et le nébuliseur est, lui, connecté à une pièce en Y. Il s'en suit pour le praticien l'obligation de connecter/déconnecter tour à tour ces différents éléments sur le circuit de ventilation.

On connaît aussi des dispositifs de ventilation mécanique incluant sur le trajet du flux d'air entre le ventilateur, qui dispense l'air, et la sonde d'intubation, qui apporte l'air au patient, une chambre d'inhalation, parfois appelée espaceur, sur laquelle il est possible de brancher à la fois un nébuliseur et un aérosol doseur. Un tel dispositif est par exemple décrit dans les demande des brevets WO 01/83011, US 6962152 B1, WO 2010/100557 A1 et US 2008/0264412 A1. La
chambre d'inhalation décrite dans cette demande de brevet se présente sous la forme d'un ballon rigide de forme ovale et comporte une entrée et une sortie d'air disposées en vis à vis dans l'axe longitudinal médian séparant la chambre en deux parties, une partie supérieure et une partie inférieure. L'entrée et la sortie d'air sont adaptées aux tubages d'un respirateur ou d'un système de ventilation mécanique. La chambre comprend encore deux ouvertures prévues pour connecter un aérosol-doseur et un nébuliseur, respectivement. L'ouverture prévue pour connecter l'aérosol doseur est située dans la partie supérieure de la chambre alors que celle prévue pour connecter le nébuliseur est située dans la partie inférieure. Les ouvertures destinées au nébuliseur et à l'aérosol doseur sont en vis à vis.

Un tel dispositif n'est pas entièrement satisfaisant. En effet, les particules délivrées par les nébuliseurs ont, d'une manière générale, une forte tendance à sédimenter dans la chambre d'inhalation, ce phénomène étant accentué par la disposition du nébuliseur dans la partie inférieure de la chambre au cours de son utilisation.

Le brevet US 5 178 138 décrit un système de ventilation mécanique sur lequel il est prévu de connecter à la fois un nébuliseur et un aérosol doseur. Le système de ventilation inclut une chambre d'inhalation sur laquelle est connecté l'aérosol doseur. Le nébuliseur n'est pas connecté sur la chambre d'inhalation mais sur une pièce en T elle-même raccordée à la chambre d'inhalation.

Ce type de dispositif n'est pas non plus optimum. En effet, le nébuliseur génère un flux de particules selon un axe vertical et la distance de freinage des particules dans ce dispositif ne permet pas d'éviter le dépôt de celles-ci par impaction sur les parois de la pièce en T.

De même, les dispositifs de l'état de la technique ne permettent pas non plus d'optimiser la délivrance des substances contenues dans l'aérosol-doseur. En effet, celui-ci génère un flux selon un axe de projection horizontal. Il convient alors de lui octroyer une distance de freinage importante afin d'éviter le dépôt des particules par impaction sur les parois de la chambre.

De plus, la ventilation de la chambre par l'air en provenance du respirateur permet le transport de l'aérosol hors de la chambre. La forme de la chambre est donc un élément important à prendre en compte afin d'éviter qu'elle ne contienne des zones mal ventilées dans lesquelles l'air re-circule.

Le but de la présente invention est de proposer un dispositif de ventilation mécanique incluant une chambre d'inhalation qui permette la délivrance de substances médicales à la fois par nébuliseur et par aérosol-doseur et qui limite, voire qui supprime, les inconvénients précités, en particulier les dépôts par impaction et/ou par sédimentation des substances dans la chambre d'inhalation.

A cet effet, l'invention concerne une chambre d'inhalation destinée à être intégrée sur un circuit d'un dispositif de respiration en ventilation mécanique et à être traversée par un flux gazeux, ladite chambre d'inhalation comprenant deux parties de part et d'autre de son axe longitudinal et comportant des ouvertures débouchant dans son volume intérieur, dont deux ouvertures destinées à être connectées avec le circuit du respirateur, l'une pour l'entrée d'un flux gazeux, l'autre pour la sortie dudit flux gazeux, ainsi qu'une ouverture destinée à recevoir un aérosol doseur pressurisé, et une ouverture destinée à recevoir un nébuliseur, la chambre d'inhalation se caractérisant en ce que les ouvertures destinées à recevoir l'aérosol doseur et le nébuliseur sont toutes deux pratiquées dans la même partie de ladite chambre d'inhalation par rapport à l'axe longitudinal.

La disposition des ouvertures destinées à recevoir l'aérosol doseur et le nébuliseur, non en vis à vis mais l'une adjacente à l'autre, permet d'éviter le dépôt par impaction des particules, par exemple produites par le nébuliseur, sur l'aérosol doseur ou inversement.

La chambre d'inhalation selon l'invention permet également de connecter à la fois un aérosol doseur et un nébuliseur. Cela permet une simplification de la pratique clinique puisqu'il n'est pas nécessaire de déconnecter l'un pour utiliser l'autre. Il s'en suit un gain de temps pour le praticien ainsi qu'une diminution des risques pour le patient.

Selon une caractéristique de l'invention, la partie commune dans laquelle les ouvertures destinées à recevoir l'aérosol doseur et le nébuliseur sont pratiquées est la partie dite supérieure de la chambre lorsque celle-ci est intégrée sur un circuit d'un dispositif de respiration en ventilation mécanique en cours de fonctionnement.

Lorsque le nébuliseur est connecté à la chambre d'inhalation dans sa partie supérieure, c'est à dire sur le dessus de la chambre lorsque celle-ci est utilisée, les particules introduites disposent alors d'un temps de sédimentation plus important que si elles étaient introduites par le dessous de la chambre comme cela est le cas avec les chambres d'inhalation de l'état de la technique, ce qui permet de limiter la perte par sédimentation.

Au sens de l'invention, on entend par «partie supérieure de la chambre », la partie de la chambre se trouvant au-dessus de l'axe longitudinal de la chambre lorsqu'elle est utilisée dans un dispositif de ventilation mécanique ou encore la partie se trouvant au dessus-du flux gazeux traversant la chambre lorsque la chambre est utilisée dans un dispositif de ventilation mécanique en cours de fonctionnement.

Avantageusement, la chambre d'inhalation comporte encore deux portions tronconiques dont les grandes bases sont communes, les petites bases étant prolongées de portions cylindriques, l'une de ces portions cylindriques étant en communication avec l'entrée prévue pour le flux gazeux provenant du dispositif de ventilation, l'autre étant en communication avec la sortie prévue pour le flux gazeux.

La forme de la chambre d'inhalation joue un rôle dans le dépôt des particules sur les parois internes. Une chambre de forme cylindrique, par exemple, a pour effet d'augmenter ce phénomène résultant de la mauvaise ventilation de la chambre. La forme de la chambre selon l'invention est particulièrement adaptée à la diffusion d'aérosol et limite le dépôt par impaction. L'utilisation de formes coniques ou équivalentes permet d'assurer un bon transport de l'aérosol hors de la chambre et de limiter les zones de re-circulation de l'air, c'est-à-dire les zones provoquant une mauvaise ventilation dans la chambre.

La chambre peut être composée de deux parties tronconiques symétriques ou non symétriques.

Ainsi, selon un mode de réalisation de l'invention, la pente d'une des portions tronconiques, préférentiellement de la portion tronconique la plus proche de l'entrée prévue pour le flux gazeux, est plus forte et plus courte que celle de la portion tronconique proche de la sortie prévue pour le flux gazeux. Dans ce mode de réalisation, la portion tronconique de la chambre en communication avec l'entrée d'air est moins volumineuse que la portion tronconique en communication avec la sortie d'air lorsque la chambre est connectée au dispositif de ventilation en fonctionnement.

Avantageusement, l'ouverture prévue pour recevoir le nébuliseur se trouve dans la section la plus large de la chambre d'inhalation. Préférentiellement, l'ouverture destinée à recevoir le nébuliseur est située à une distance d'au moins 4 cm de la paroi de la chambre lui faisant face.

Lorsque le nébuliseur est connecté à la chambre d'inhalation dans sa partie supérieure et dans sa section la plus large, les particules introduites selon un axe de projection vertical disposent d'une distance de freinage permettant de limiter ou d'éviter leur dépôt par impaction sur les parois de la chambre. De même, cette configuration permet de limiter encore le dépôt par sédimentation en augmentant le temps de sédimentation.

Selon un mode de réalisation de l'invention, l'axe de l'ouverture prévue pour recevoir le nébuliseur forme un angle inférieur ou égal à 90° avec l'axe longitudinal de la chambre d'inhalation.

Selon un mode de réalisation de l'invention, l'ouverture prévue pour recevoir l'aérosol doseur se trouve dans une section la plus étroite de la chambre. Préférentiellement elle se trouve aménagée dans la portion cylindrique de la chambre en communication avec l'entrée prévue pour le flux gazeux provenant du dispositif de ventilation.

Cette configuration offre aux flux de particules émises par l'aérosol doseur selon un axe de projection horizontal une distance de freinage permettant de limiter ou de supprimer leur dépôt par impaction.

Selon un mode de réalisation préféré de l'invention, l'ouverture prévue pour recevoir le nébuliseur se trouve en aval de l'ouverture prévue pour recevoir l'aérosol doseur par rapport à l'entrée du flux gazeux, lorsque la chambre d'inhalation est connectée sur le circuit d'un dispositif de ventilation mécanique.

Préférentiellement, l'ouverture prévue pour recevoir l'aérosol doseur comprend un moyen prévu pour orienter le flux de l'aérosol selon un axe horizontal à l'axe longitudinal de la chambre d'inhalation.

L'ouverture destinée à recevoir l'aérosol doseur est munie d'un moyen tel qu'un gicleur créant un obstacle dans la chambre de stockage. Le nébuliseur produit des particules liquides susceptibles de s'impacter lorsque les particules rencontrent un obstacle. Lorsque le nébuliseur est placé en amont de l'ouverture du pMDI, les particules peuvent s'impacter sur le gicleur et obstruer celui-ci, puisque l'air produit par le respirateur traverse la chambre de manière unidirectionnelle, rendant ainsi impossible son utilisation avec le pMDI. La solution proposée pour remédier à cet inconvénient consiste donc à placer le nébuliseur en aval du pMDI par rapport au flux gazeux traversant la chambre.

Avantageusement, les ouvertures destinées à recevoir le nébuliseur et l'aérosol doseur comportent, respectivement, un moyen d'obturation.

En effet, lorsque seul un des dispositifs, soit le nébuliseur, soit l'aérosol doseur, est connecté à la chambre d'inhalation il est nécessaire de fermer l'ouverture destinée à recevoir l'autre dispositif par un moyen étanche.

Selon une caractéristique de l'invention, le volume intérieur de la chambre d'inhalation est inférieur à 500 mL, préférentiellement inférieur à 300 mL. En effet, la chambre d'inhalation doit avoir un volume intérieur inférieur au volume inspiratoire du patient lequel correspond à 500 mL de manière standard.

Plus précisément encore, le volume intérieur de la chambre est inférieur au volume alvéolaire lequel se définit par la partie du volume inspiré par le patient qui va dans les poumons, site cible pour le médicament (volume inspiré - volume mort). Le volume de la chambre doit donc être préférentiellement inférieur à 300 mL.

Par ailleurs, le volume de la chambre doit être supérieur à 20 mL afin d'assurer le stockage et le freinage des particules reçues.

Ainsi, le volume intérieur de la chambre sera plus préférentiellement compris entre 20 mL et 300 mL.

L'invention concerne encore un dispositif de respiration en ventilation mécanique comportant :
- un respirateur pour insuffler un volume gazeux,
- au moins un conduit d'inhalation destiné à être emprunté par un flux gazeux lors d'une phase d'inspiration,
- au moins un conduit d'exhalation destiné à être emprunté par un flux gazeux lors d'une phase d'expiration,
- un conduit d'amenée du flux gazeux au patient,
le dispositif étant caractérisé en ce qu'il comprend une chambre d'inhalation telle que décrite dans ce qui précède.

Dans un tel dispositif, bien connu de l'homme du métier, le respirateur fonctionne comme la source d'un gaz, généralement de l'air et/ou de l'oxygène, et comprend une unité permettant le contrôle des paramètres de la ventilation. Il permet, notamment, de pouvoir contrôler les phases inspiratoires et expiratoires.

Selon l'invention, la chambre d'inhalation se trouve sur le trajet du flux gazeux emprunté lors d'une phase d'inspiration ou d'inhalation.

Selon l'invention encore, l'entrée de flux gazeux de la chambre d'inhalation est connectée au conduit d'inhalation et la sortie de flux gazeux de la chambre d'inhalation est connectée au conduit d'amenée du flux gazeux au patient par l'intermédiaire d'une pièce en Y.

Avantageusement, la chambre d'inhalation, le conduit d'exhalation et le conduit d'amené au patient sont respectivement connectés à la pièce en Y.

Selon un mode de réalisation de l'invention, le conduit d'amenée au patient est une sonde d'intubation.

Selon un autre mode de réalisation de l'invention, le conduit d'amenée au patient est connecté à un masque destiné à être porté par un patient.

Les caractéristiques de l'invention mentionnées ci-dessus, ainsi que d'autres, apparaîtront plus clairement à la lecture de la description suivante d'exemples de réalisation, ladite description étant faite en relation avec les dessins joints, parmi lesquels :
La Fig. 1 est une vue en coupe de profile d'une chambre d'inhalation selon un premier mode de réalisation de l'invention,
La Fig. 2 est une vue en coupe de profile d'une chambre d'inhalation selon un deuxième mode de réalisation de l'invention,
La Fig. 3 est une représentation schématique d'un dispositif de respiration en ventilation mécanique comportant une chambre d'inhalation selon l'invention,
La Fig. 4 est une représentation schématique d'un dispositif de respiration en ventilation mécanique, en cours de fonctionnement, comportant une chambre d'inhalation selon l'invention sur laquelle est connecté un nébuliseur, au cours d'une phase d'expiration,
La Fig. 5 est une représentation schématique d'un dispositif de respiration en ventilation mécanique, en cours de fonctionnement, comportant une chambre d'inhalation selon l'invention sur laquelle est connecté un nébuliseur, au cours d'une phase d'inspiration,
La Fig. 6 est une représentation schématique d'un dispositif de respiration en ventilation mécanique, en cours de fonctionnement, comportant une chambre d'inhalation selon l'invention sur laquelle est connecté un aérosol doseur, au cours d'une phase d'expiration,
La Fig. 7 est une représentation schématique d'un dispositif de respiration en ventilation mécanique, en cours de fonctionnement, comportant une chambre d'inhalation selon l'invention sur laquelle est connecté un aérosol doseur, au cours d'une phase d'inspiration.

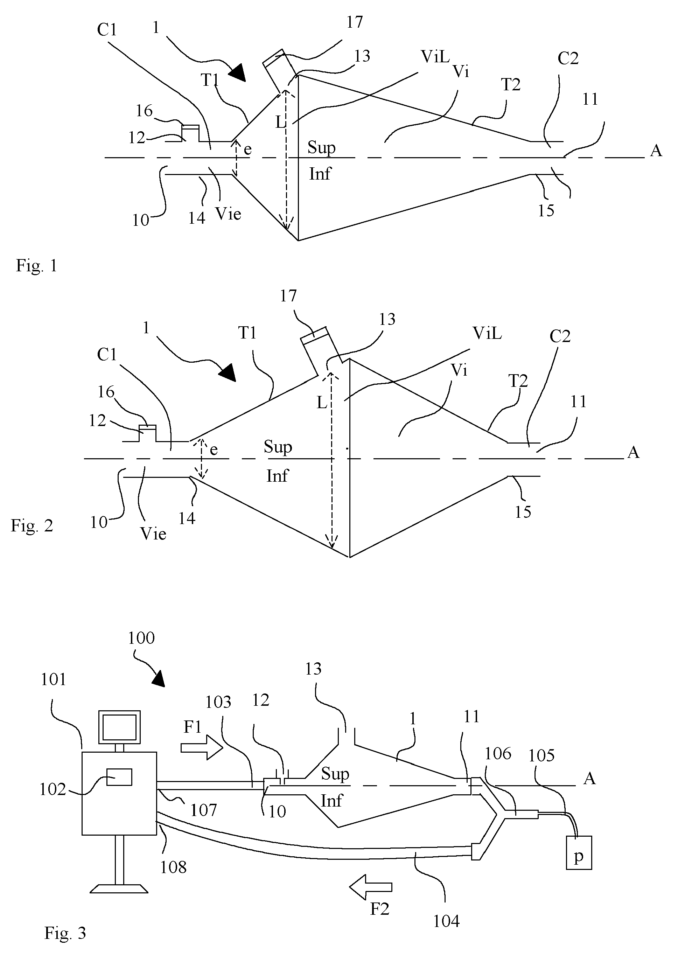

En lien avec la Fig. 1 est illustrée une chambre d'inhalation 1 selon un premier mode de réalisation de l'invention. La chambre d'inhalation 1 est par exemple, composée en polycarbonates, et définit un volume intérieur Vi destiné à recevoir les particules provenant d'un nébuliseur et/ou d'un aérosol doseur et à être traversé par un flux gazeux généré par un dispositif en ventilation mécanique.

La chambre d'inhalation 1 comporte quatre ouvertures. La première ouverture (10) est une entrée pour un flux gazeux émis par un dispositif de ventilation mécanique, tel que de l'air, la seconde est une sortie (11) pour ce même flux. Ces deux ouvertures sont pratiquées dans le même axe longitudinal A de la chambre d'inhalation 1. Les dimensions des ouvertures 10 et 11 sont adaptées pour permettre la connexion de la chambre 1 sur les tubages d'un dispositif de ventilation mécanique conventionnel. La troisième est une ouverture 12 destinée à recevoir un aérosol doseur pressurisé (pMDI), et la quatrième est une ouverture 13 destinée à recevoir un nébuliseur, préférentiellement à tamis vibrant.

L'axe longitudinal A sépare la chambre d'inhalation 1 en deux parties. Les ouvertures 12 et 13 sont aménagées dans une partie commune de la chambre d'inhalation 1. Lorsque la chambre est incorporée à un dispositif de ventilation mécanique, la partie commune comprenant les ouvertures 12 et 13 est la partie supérieure Sup de la chambre par opposition à la partie inférieure Inf.

La chambre d'inhalation 1 est encore composée de deux portions tronconiques à grandes bases communes T1, T2. Cela permet de définir une section large L de la chambre 1 dans une région centrale de la chambre 1 où le volume intérieur Vi est le plus important ainsi que deux sections étroites où le volume intérieur Vi est plus restreint à proximité de l'entrée 10 et de la sortie 11, respectivement. Les petites bases de la chambre d'inhalation 1 sont prolongées de portions cylindriques 14, 15, la portion cylindrique 14 étant en communication avec l'entrée 10 du flux gazeux et la portion cylindrique 15 étant en communication avec la sortie 11 du flux gazeux.

La pente de la partie tronconique T1 débouchant sur l'entrée 10 pour le flux gazeux est plus forte et courte que celle de la partie tronconique T2 débouchant sur la sortie 11 du flux gazeux.

L'ouverture 13 pour le nébuliseur est pratiquée dans la section la plus large L de la chambre 1 de manière à ce que les particules émises débouchent là où le volume intérieur Vi est le plus important et noté ViL. L'axe de l'ouverture 13 forme un angle inférieur ou égal à 90° avec l'axe longitudinal A de la chambre d'inhalation.

L'ouverture 12 prévue pour recevoir l'aérosol doseur se trouve aménagée, elle, dans une section étroite e de la chambre 1, préférentiellement dans la portion cylindrique C1 en communication avec l'entrée 10 pour flux gazeux. Les particules émises débouchent donc dans ce cas là où le volume intérieur Vi est le moins important, noté Vie. Le volume Vie est inférieur au volume ViL. En outre, l'ouverture 12 est située en amont de l'ouverture 13 ce qui permet d'éviter que les particules émises par un nébuliseur ne viennent obstruer par impaction l'orifice de sortie de l'aérosol doseur lorsque celui-ci est connecté. Ainsi l'ouverture 13 est ici placée sur la partie tronconique T1 mais pourrait aussi être placée sur la partie tronconique T2.

Les ouvertures 12 et 13 comportent respectivement des moyens d'obturation 16, 17 prévus pour les fermer de manière étanche lorsqu'aucun nébuliseur et/ou aérosol doseur n'est connecté à la chambre d'inhalation 1.

La chambre d'inhalation 1 représentée sur la Fig. 2 comporte les mêmes caractéristiques que la chambre d'inhalation 1 présentée sur les Figs. 1 et 3 à 7, à l'exception des portions tronconiques T1, T2 qui sont, selon cette variante de l'invention, symétriques, les pentes des parties tronconiques T1, T2 étant identiques.

En lien avec la Fig. 3 est représenté un dispositif de respiration en ventilation mécanique 100 dans lequel est intégrée une chambre d'inhalation 1 selon l'invention.

Le dispositif 100 comprend, de manière classique, un respirateur 101 pour insuffler un volume gazeux à un patient P. Le respirateur 101 est muni d'une unité 102 apte à contrôler le débit et la pression du gaz, généralement un mélange air/oxygène.

Le dispositif 100 comprend encore un conduit d'inspiration, ou d'inhalation, 103 destiné à être emprunté par le flux gazeux lors d'une phase d'inspiration, ou d'inhalation, un conduit d'expiration 104, ou d'exhalation, destiné à être emprunté par le flux gazeux lors d'une phase d'expiration (exhalation) ainsi qu'un conduit d'amenée 105 du flux gazeux au patient P.

Le conduit d'inspiration 103 est connecté d'une part à une sortie 107 du respirateur 101 et, d'autre part, à l'entrée 10 de la chambre d'inhalation 1. La sortie 11 de la chambre 1 est, elle, connectée à une pièce en Y 106, de sorte que la chambre d'inhalation 1 se trouve sur le trajet emprunté par le flux gazeux lors des phases d'inspiration.

Une autre branche de la pièce en Y 106 est reliée au conduit 105 connecté au patient P. A ce titre, ce conduit 105 peut être remplacé par un masque porté par le patient P, selon les cas.

Le conduit d'expiration 104 est connecté d'une part, à la troisième branche de la pièce en Y 106 et, d'autre part, à une entrée 108 du respirateur 101.

La chambre d'inhalation 1 est reliée au dispositif 100 de manière à ce que les ouvertures 12 et 13 destinées à connecter, respectivement, un nébuliseur et un aérosol doseur soient sur le dessus de la chambre 1, c'est à dire dans sa partie supérieure Sup séparée de la partie inférieure Inf par l'axe longitudinal A de la chambre 1.

Le dispositif 100 définit ainsi un circuit pour un flux gazeux. Les flèches F1 et F2 représentent respectivement la circulation du flux gazeux lors d'une phase d'inspiration et lors d'une phase d'expiration.

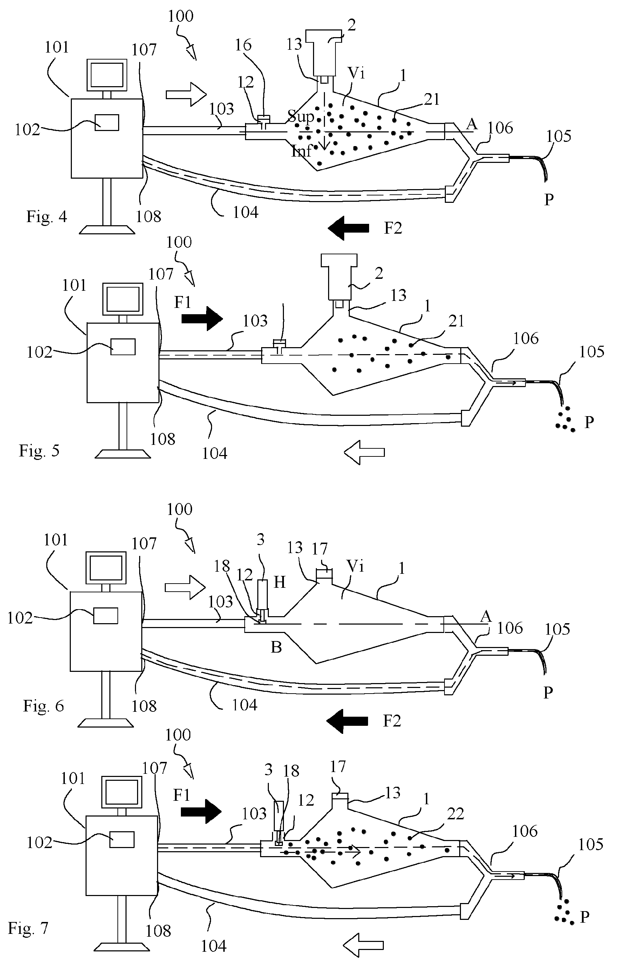

En lien avec les Figs. 4 et 5 est expliqué le fonctionnement de la chambre d'inhalation 1 dans un dispositif de ventilation mécanique 100 lorsqu'un nébuliseur 2, tel qu'un nébuliseur à tamis vibrant, est utilisé et ainsi connecté à la chambre d'inhalation 1 dans sa partie supérieure Sup.

La Fig. 4 représente une phase d'expiration d'air par le patient P. Le flux gazeux expiré par le patient traverse le conduit 105, emprunte la pièce en Y 106 puis le conduit d'expiration 104 (flèche F2). L'aérosol produit par le nébuliseur 2 est stocké dans la chambre d'inhalation 1. Pour ce faire, le nébuliseur 2 est actionné et les particules nébulisées pénètrent dans le volume intérieur Vi de la chambre 1 selon un axe de projection vertical. Les particules étant projetées dans la section la plus grande de la chambre, elles disposent d'un volume intérieur ViL suffisant pour limiter leur dépôt par impaction contre les parois de la chambre ou leur sédimentation.

Lors de la phase d'inspiration suivante (Fig. 5), le flux gazeux (Flèche F1) emprunte le conduit d'inspiration 103 depuis le respirateur 101 puis traverse la chambre d'inhalation 1 et entraîne avec lui les particules nébulisées 21 en direction du patient P par l'intermédiaire de la pièce en Y 106 et du tube 105.

L'ouverture 12 destinée à recevoir l'aérosol doseur est fermée par un moyen de fermeture 16 étanche.

En lien avec les Figs. 6 et 7 est expliqué maintenant le fonctionnement de la chambre d'inhalation 1 dans le dispositif de ventilation mécanique 100 lorsqu'un aérosol doseur 3 est utilisé et ainsi connecté à la chambre d'inhalation 1 sur l'ouverture 12.

La Fig. 6 représente une phase d'expiration d'air par le patient P. Lors de cette phase d'expiration, tout se passe comme expliqué en lien avec la Fig. 4 à la différence qu'aucune particule produite par l'aérosol doseur 3 n'est stockée dans la chambre d'inhalation 1.

Dès le début de la phase d'inspiration suivante, l'aérosol doseur 3 est manuellement déclenché par un praticien. Après actionnement, les particules pénètrent dans le volume intérieur Vi de la chambre 1 selon un axe de projection horizontal. En effet, l'ouverture 12 est munie d'un moyen prévu pour orienter le flux de l'aérosol selon un axe horizontal à l'axe A de la chambre d'inhalation 18, tel qu'un gicleur.

Les particules étant projetées selon un axe horizontal, donc parallèle à l'axe longitudinal A de la chambre 1, dans une section étroite e de la chambre 1 et proche de l'entrée 10 du flux gazeux, elles disposent d'un volume intérieur Vi suffisant pour limiter leur dépôt par impaction contre les parois de la chambre ou leur sédimentation.

Le flux gazeux généré par le respirateur 101 emprunte le conduit d'inspiration 103 puis traverse la chambre d'inhalation 1 en entraînant avec lui les particules 22 en direction du patient P par l'intermédiaire de la pièce en Y 106 et du tube 105.

Il est tout à fait possible d'utiliser simultanément l'aérosol doseur et le nébuliseur au cours d'un même cycle respiratoire. Du fait que le nébuliseur se trouve disposé en aval de l'aérosol doseur, les particules produites par nébulisation ne peuvent s'impacter sur l'aérosol doseur.

De même, le fonctionnement de la chambre d'inhalation 1 décrite en lien avec la Fig. 2 dans un dispositif de respiration en ventilation mécanique est identique à ce qui a été décrit en lien avec les Figs. 4 à 7.

Ainsi l'invention est particulièrement avantageuse, car elle permet de simplifier la pratique clinique du praticien et de limiter les risques induits par l'intervention sur le circuit du patient pour connecter le nébuliseur et l'aérosol doseur. L'invention permet également d'augmenter les performances des aérosols doseurs et des nébuliseurs, améliorant ainsi l'efficacité du traitement. De plus, l'invention est économiquement intéressante puisqu'elle permet d'une part d'administrer une plus grande quantité de médicament au patient et d'autre part de réduire le coût des connectiques par l'utilisation d'une unique connectique en comparaison des deux actuellement utilisées pour l'aérosol doseur et le nébuliseur.

## Revendications

1. Chambre d'inhalation (1) destinée à être intégrée sur un circuit d'un dispositif de respiration en ventilation mécanique (100) et à être traversée par un flux gazeux, ladite chambre d'inhalation (1) étant composée de deux parties de part et d'autre de son axe longitudinal (A) et comportant quatre ouvertures débouchant dans son volume intérieur (Vi), dont deux ouvertures (10, 11) destinées à être connectées avec le circuit du respirateur, l'une (10) pour l'entrée du flux gazeux, l'autre (11) pour la sortie du flux gazeux, une ouverture (12) destinée à recevoir un aérosol doseur pressurisé (3) et une ouverture (13) destinée à recevoir un nébuliseur (2), la chambre présentant des sections dont une section (L) de largeur plus importante que les largeurs des autres sections et une section de largeur plus étroite (e), les ouvertures (12, 13) destinées à recevoir l'aérosol doseur (3) et le nébuliseur (2) sont toutes deux pratiquées dans la même partie de ladite chambre d'inhalation (1) par rapport à l'axe longitudinal (A), l'ouverture (13) prévue pour recevoir le nébuliseur (2) étant située dans la section la plus large (L) de la chambre d'inhalation (1) et l'ouverture (12) prévue pour recevoir l'aérosol doseur (3) étant située dans la section plus étroite (e) de la chambre d'inhalation (1), lesdites ouvertures (12, 13) destinées à recevoir le nébuliseur (2) et l'aérosol doseur (3), respectivement, comportant chacune un moyen d'obturation (17, 16).

2. Chambre d'inhalation (1) selon la revendication 1, **caractérisée en ce que** ladite partie dans laquelle les ouvertures (12, 13) destinées à recevoir l'aérosol doseur (3) et le nébuliseur (2) sont pratiquées est la partie dite supérieure (Sup) de ladite chambre (1) au-dessus du flux gazeux traversant la chambre lorsque la chambre est utilisée dans un dispositif de ventilation mécanique en cours de fonctionnement dans un circuit d'un dispositif de respiration en ventilation mécanique (100).

3. Chambre d'inhalation (1) selon la revendication 1 ou 2, **caractérisée en ce qu'**elle est, de plus, formée de deux portions tronconiques (T1, T2) dont les grandes bases sont communes, les petites bases étant prolongées de portions cylindriques (C1, C2), l'une de ces portions cylindriques (C1) étant en communication avec l'entrée (10) prévue pour le flux gazeux l'autre (C2) étant en communication avec la sortie (11) prévue pour le flux gazeux.

4. Chambre d'inhalation (1) selon l'une des revendications précédentes, **caractérisée en ce que** l'ouverture (13) prévue pour recevoir le nébuliseur (2) est située en aval de l'ouverture (12) prévue pour recevoir l'aérosol doseur (3) par rapport à l'entrée (10) du flux gazeux.

5. Chambre d'inhalation (1) selon l'une des revendications précédentes, **caractérisée en ce que** la pente de la portion tronconique (T1) la plus proche de l'entrée (10) de flux gazeux est plus forte et courte que celle de la partie tronconique (T2) la plus proche de la sortie (11) du flux gazeux.

6. Chambre d'inhalation (1) selon l'une des revendications précédentes, **caractérisée en ce que** l'axe de l'ouverture (12) prévue pour recevoir le nébuliseur (2) forme un angle inférieur ou égal à 90° avec l'axe longitudinal (A) de la chambre d'inhalation (1).

7. Chambre d'inhalation (1) selon l'une des revendications précédentes, **caractérisée en ce que** l'ouverture (13) prévue pour recevoir l'aérosol doseur (3) comprend un moyen (18) prévu pour orienter le flux de l'aérosol selon un axe horizontal à l'axe longitudinal (A) de la chambre d'inhalation (1).

8. Chambre d'inhalation (1) selon l'une des revendications précédentes, **caractérisée en ce que** ledit volume intérieur (Vi) est inférieur à 500 mL, préférentiellement inférieur à 300 mL, plus préférentiellement compris entre 20 mL et 300 mL.

9. Dispositif de respiration en ventilation mécanique (100) comportant :
- un respirateur (101) pour insuffler un volume gazeux comprenant une sortie (102) et une entrée (101) pour un flux gazeux,
- un conduit d'inhalation (103) connecté à ladite sortie (102) du flux gazeux,
- un conduit d'exhalation (104) connecté à ladite entrée (103) du flux gazeux,
- un conduit d'amenée (105) du flux gazeux au patient (P),
**caractérisé en ce qu'**il comprend une chambre d'inhalation (1) selon l'une des revendications précédentes.

10. Dispositif de respiration en ventilation mécanique (100) selon la revendication 9, **caractérisé en ce que** ladite chambre d'inhalation (1) se trouve sur le trajet du flux gazeux emprunté lors d'une phase d'inhalation.

11. Dispositif de respiration en ventilation mécanique (100) selon l'une des revendications 9 à 10, **caractérisé en ce que** l'entrée (10) de flux gazeux de ladite chambre d'inhalation (1) est connectée au conduit d'inhalation (102) et la sortie (11) de flux gazeux de ladite chambre d'inhalation (1) est connectée au conduit d'amenée (105) du flux gazeux au patient par l'intermédiaire d'une pièce en Y (106).

12. Dispositif de respiration en ventilation mécanique (100) selon l'une des revendications 9 à 11, **caractérisé en ce que** sur l'une des branches de la pièce en Y (106) est connectée la chambre d'inhalation (1), sur une autre branche est connecté le conduit d'exhalation (104) et sur encore une autre branche de la pièce en Y (106) est connecté le conduit d'amenée (105) au patient (P).

13. Dispositif de respiration en ventilation mécanique (100) selon l'une des revendications 9 à 12, **caractérisé en ce que** le conduit d'amenée (105) au patient (P) est une sonde d'intubation.

## Patentansprüche

1. Inhalatorkammer (1), die dazu bestimmt ist, in einen Kreislauf einer mechanischen Beatmungsanlage (100) integriert und von einem Gasstrom durchströmt zu werden, wobei sich die genannte Inhalatorkammer (1) aus zwei Teilen beiderseits ihrer Längsachse (A) zusammensetzt und vier, in ihr Innenvolumen (Vi) mündende Öffnungen umfasst, von denen zwei (10, 11) dazu bestimmt sind, an den Kreislauf des Respirators angeschlossen zu werden, und zwar die eine (10) für den Gasstromeintritt und die andere (11) für den Gasstromaustritt, eine Öffnung (12) zur Aufnahme eines treibgasgetriebenen Dosieraerosols (3) und eine Öffnung (13) zur Aufnahme eines Verneblers (2) bestimmt sind, wobei die Kammer Abschnitte aufweist, von denen ein Abschnitt (L) eine größere Breite aufweist, als die anderen Abschnitte, sowie eine schmalere Breite (e), die zur Aufnahme des Dosieraerosols (3) und des Vernebiers (2) bestimmten Öffnungen (12, 13) beide in Bezug auf die Längsachse in demselben Teil der genannten Inhalatorkammer (1) ausgebildet sind, sich die, für die Aufnahme des Verneblers (2) vorgesehene Öffnung (13) in dem breiteren Abschnitt (L) der Inhalatorkammer (1) und die, für die Aufnahme des Dosieraersols vorgesehene Öffnung (12) in dem schmaleren Abschnitt ( e) der Inhalatorkammer (1) befinden, wobei jede der genannten, jeweils zur Aufnahme des Verneblers (2) und des Dosieraerosols (3) bestimmten Öffnungen (12, 13) ein Verschlussmittel (17, 16) umfasst.

2. Inhalatorkammer (1) nach Patentanspruch 1, **dadurch gekennzeichnet, dass** der genannte Teil, in dem die, zur Aufnahme des Dosieraersols (3) und des Verneblers (2) bestimmten Öffnungen ausgebildet sind, der sogenannte obere Teil (Sup) der genannten Kammer (1) ist, der oberhalb des, die Kammer durchströmenden Gasstroms liegt, wenn diese in einer mechanischen Beatmungsvorrichtung verwendet wird, die in einem Kreislauf einer mechanischen Beatmungsanlage (100) in Betrieb steht.

3. Inhalatorkammer (1) nach Patentanspruch 1 oder 2, **dadurch gekennzeichnet, dass** sie zudem durch zwei kegelstumpfförmige Teile (T1, T2 gebildet ist, die eine gemeinsame große Grundfläche haben, wobei die kleinen Grundflächen durch zylindrische Teile (C1, C2) verlängert sind, eines dieser zylindrischen Teile (C1) mit dem, für den Gasstrom vorgesehenen Eintritt (10), das andere (C2) mit dem für den Gasstrom vorgesehenen Austritt (11) in Verbindung stehen.

4. Inhalatorkammer (1) nach einem der vorstehenden Patentansprüche, **dadurch gekennzeichnet, dass** die, zur Aufnahme des Verneblers (2) vorgesehene Öffnung (13) in Bezug auf den Eintritt des Gasstroms (10) der, zur Aufnahme des Dosieraersols vorgesehenen Öffnung (12) nachgelagert ist.

5. Inhalatorkammer (1) nach einem der vorstehenden Patentansprüche, **dadurch gekennzeichnet, dass** die Schräge des, dem Gasstromeintritt (10) am nächsten gelegenen kegelstumpfförmigen Teils (T1) stärker und kürzer ist, als die des kegelstumpfförmigen Teils (T2), das dem Gasstromaustritt (11) am nächsten gelegen ist.

6. Inhalatorkammer (1) nach einem der vorstehenden Patentansprüche, **dadurch gekennzeichnet, dass** die Achse der zur Aufnahme des Verneblers (2) vorgesehenen Öffnung (12) in einem Winkel zur Längsachse (A) der Inhalatorkammer (1) steht, der kleiner oder gleich 90° ist.

7. Inhalatorkammer (1) nach einem der vorstehenden Patentansprüche, **dadurch gekennzeichnet, dass** die, zur Aufnahme des Dosieraersols (3) vorgesehene Öffnung (13) ein Mittel (18) umfasst, dass vorgesehen ist, um den Aerosolstrom entlang einer, horizontal zur Längsachse (A) der Inhalatorkammer (1) verlaufenden Achse zu leiten.

8. Inhalatorkammer (1) nach einem der vorstehenden Patentansprüche, **dadurch gekennzeichnet, dass** das genannte Innenvolumen (Vi) weniger als 500 ml, vorzugsweise weniger als 300 ml, ganz bevorzugt zwischen 20 ml und 300ml beträgt.

9. Mechanische Beatmungsanlage (100) umfassend:
- einen Respirator (101) zum Einblasen eines Gasvolumens umfassend einen Austritt (102) und einen Eintritt (101) für einen Gasstrom,
- eine Inhalationsleitung (103), die an den genannten Austritt (102) des Gastroms angeschlossen ist,
- eine Exhalationsleitung (104), die an den genannten Eintritt (103) des Gasstroms angeschlossen ist,
- eine Zufuhrleitung (105) für die Zufuhr des Gasstroms zum Patienten (P),
**dadurch gekennzeichnet, dass** die Anlage eine Inhalatorkammer (1) nach einem der vorstehenden Patentansprüche umfasst.

10. Mechanische Beatmungsanlage (100) nach Patentanspruch 9, **dadurch gekennzeichnet, dass** sich die genannte Inhalatorkammer (1) im Strömungsweg des Gasstroms befindet, den dieser während einer Inhalationsphase durchströmt.

11. Mechanische Beatmungsanlage (100) nach einem der Patentansprüche 9 bis 10, **dadurch gekennzeichnet, dass** der Gasstromeintritt (10) der genannten Inhalatorkammer (1) an die Inhalationsleitung (102) angeschlossen ist und der Gasstromaustritt der genannten Inhalationskammer (1) über ein Y-förmiges Teil (106) an die Zufuhrleitung (105) des Gasstroms zum Patienten angeschlossen ist.

12. Mechanische Beatmungsanlage (100) nach einem der Patentansprüche 9 bis 11, **dadurch gekennzeichnet, dass** einer der Äste des Y-förmigen Teils (106) an die Inhalatorkammer, die Exhalationsleitung (104) an einen anderen Ast und die Zufuhrleitung (105) zum Patienten (P) an noch einem anderen Ast des Y-förmigen Teils (106) angeschlossen ist.

13. Mechanische Beatmungsanlage (100) nach einem der Patentansprüche 9 bis 12, **dadurch gekennzeichnet, dass** die Zufuhrleitung (105) zum Patienten (P) eine Intubationssonde ist.

## Claims

1. Inhalation chamber (1) intended to be incorporated into a circuit of a mechanical-ventilation respiration device (100) and to be passed through by a gas stream, said inhalation chamber (1) being made up of two parts on either side of its longitudinal axis (A) and comprising four openings that lead into its internal volume (Vi), two (10, 11) of said openings being intended to be connected to the circuit of the respirator, one (10) for the inlet of the gas stream and the other (11) for the outlet of the gas stream, one (12) of said openings being intended to hold a pressurized metered-dose inhaler (3) and one (13) of said openings being' intended to hold a nebulizer (2), the chamber having sections of which one section (L) has a width greater than the widths of the other sections and one section has a narrower width (e), the openings (12, 13) intended to hold the metered-dose inhaler (3) and the nebulizer (2) both being made in the same part of said inhalation chamber (1) with respect to the longitudinal axis (A), the opening (13) provided to hold the nebulizer (2) being situated in the wider section (L) of the inhalation chamber (1) and the opening (12) provided to hold the metered-dose inhaler (3) being situated in the narrower section (e) of the inhalation chamber (1), said openings (12, 13) that are intended to hold the nebulizer (2) and the metered-dose inhaler (3), respectively, each having a closure means (17, 16).

2. Inhalation chamber (1) according to Claim 1, **characterized in that** said part in which the openings (12, 13) that are intended to hold the metered-dose inhaler (3) and the nebulizer (2) are made is the part known as the upper part (Sup) of said chamber (1) above the gas stream passing through the chamber when the chamber is used in a mechanical ventilation device during operation in a circuit of a mechanical-ventilation respiration device (100).

3. Inhalation chamber (1) according to Claim 1 or 2, **characterized in that** it is additionally formed of two frustoconical portions (T1, T2), the large bases of which are shared, the small bases being extended by cylindrical portions (C1, C2), one of these cylindrical portions (C1) being in communication with the inlet (10) provided for the gas stream and the other (C2) being in communication with the outlet (11) provided for the gas stream.

4. Inhalation chamber (1) according to one of the preceding claims, **characterized in that** the opening (13) provided for holding the nebulizer (2) is situated downstream of the opening (12) provided to hold the metered-dose inhaler (3) with respect to the gas stream inlet (10).

5. Inhalation chamber (1) according to one of the preceding claims, **characterized in that** the gradient of the frustoconical portion (T1) closest to the gas stream inlet (10) is steeper and shorter than that of the frustoconical part (T2) closest to the gas stream outlet (11).

6. Inhalation chamber (1) according to one of the preceding claims, **characterized in that** the axis of the opening (12) provided to hold the nebulizer (2) forms an angle of less than or equal to 90° with the longitudinal axis (A) of the inhalation chamber (1).

7. Inhalation chamber (1) according to one of the preceding claims, **characterized in that** the opening (13) provided to hold the metered-dose inhaler (3) comprises a means (18) provided to orient the stream of aerosol along an axis horizontal to the longitudinal axis (A) of the inhalation chamber (1).

8. Inhalation chamber (1) according to one of the preceding claims, **characterized in that** said internal volume (Vi) is less than 500 mL, preferably less than 300 mL, more preferably between 20 mL and 300 mL.

9. Mechanical-ventilation respiration device (100) comprising:
- a respirator (101) for blowing in a gaseous volume, comprising an outlet (102) and an inlet (101) for a gas stream,
- an inhalation duct (103) connected to said gas stream outlet (102),
- an exhalation duct (104) connected to said gas stream inlet (103),
- a supply duct (105) for supplying the gas stream to the patient (P),
**characterized in that** it comprises an inhalation chamber (1) according to one of the preceding claims.

10. Mechanical-ventilation respiration device (100) according to Claim 9, **characterized in that** said inhalation chamber (1) is located on the path of the gas stream that the latter takes during an inhalation phase.

11. Mechanical-ventilation respiration device (100) according to either of Claims 9 and 10, **characterized in that** the gas stream inlet (10) of said inhalation chamber (1) is connected to the inhalation duct (102) and the gas stream outlet (11) of said inhalation chamber (1) is connected to the supply duct (105) for supplying the gas stream to the patient by way of a Y-shaped part (106).

12. Mechanical-ventilation respiration device (100) according to one of Claims 9 to 11, **characterized in that** the inhalation chamber (1) is connected to one of the arms of the Y-shaped part (106), the exhalation duct (104) is connected to another arm, and the supply duct (105) to the patient (P) is connected to yet another arm of the Y-shaped part (106).

13. Mechanical-ventilation respiration device (100) according to one of Claims 9 to 12, **characterized in that** the supply duct (105) to the patient (P) is an endotracheal tube.
